# EUROPEAN PATENT SPECIFICATION

(11) **EP 2 296 608 B1**
(45) Date of publication and mention of the grant of the patent: **22.04.2015**
(21) Application number: 09769098.6
(22) Date of filing: 02.06.2009
(51) Int. Cl.: A61K 8/41, A61K 8/81, A61K 8/02, A61Q 5/00, A61K 8/03, A61Q 5/12

(54) **PERSONAL CARE COMPOSITION**
KÖRPERPFLEGEZUSAMMENSETZUNG
COMPOSITION DE SOINS PERSONNELS

(30) Priority: 27.06.2008 EP 08159261
(43) Date of publication of application: 23.03.2011
(73) Proprietor: Unilever PLC, A Company Registered in England and Wales under Company no. 41424, London EC4Y 0DY (GB); Unilever N.V., 3013 AL Rotterdam (NL)
(72) Inventor: IVANOVA, Katya, Merseyside CH63 3JW (GB); SHAW, Hannah, Elizabeth, Notthingham NG11 8BX (GB)
(74) Representative: Tansley, Sally Elizabeth
(86) International application number: PCT/EP2009/056723
(87) International publication number: WO 2009/156249

(56) References cited:
- US-A1- 2006 078 527
- US-A1- 2006 079 422

## Description

The present invention relates to multi-phase personal care compositions, more particularly hair care compositions.

Hair care compositions that have more than one phase are seen as attractive by the consumer, especially dual phase products having one transparent phase. Conditioning formulations are, in general, non transparent and often packed into a non transparent packaging as well and, therefore, lacking the attractiveness, based on appearance judgment, to the consumer.

However there are difficulties in formulating such dual phase products, in that many ingredients that are used in hair conditioners are incompatible with the rheology modifiers which can be used to prepare such a product. Even if incompatible ingredients are kept in separate phases problems occur at the junction of the phases.

US 20040234491 relates to the use of a cationic rheology modifier polyquaternium 37 in a transparent hair conditioning composition.

US2006/0079422 discloses dual phase personal care composition comprising a cleansing phase and a benefit phase.

The present invention relates to a stable dual phase product that is can be co-extruded during its manufacture into the pack, and when dispensed from the pack.

Accordingly the present invention relates to a multi-phase personal care composition comprising at least two visually distinct phases in physical contact with one another characterised in that at least one phase comprises polyacrylate-1 crosspolymer and one phase is a conditioning gel phase.

Further described is a pack comprising a multi-phase composition as described above.

In the context of the present invention the term "visually distinct," means that the regions occupied by each phase can be separately seen by the human eye as distinctly separate regions in contact with one another (i.e. they are not emulsions or dispersions of particles of less than about 100 microns).

The term "multi-phased" or "multi-phase" as used herein, is meant that at least two phases occupy separate and distinct physical spaces inside the package in which they are stored, but are in direct contact with one another (i.e., they are not separated by a barrier).

In one preferred embodiment of the present invention, the "multi-phased" personal care compositions comprising at least two phases are present within the container as a visually distinct pattern. The pattern results from the mixing or homogenization of the "multi-phased" composition. The phases may be various different colours, or include particles, glitter or pearlescence.

In the context of present invention the term "visibly clear" means that the transmission of the composition is greater than 60%, preferably greater than 80%. The transparency of the composition is measured using Ultra-Violet/Visible (UV/VIS) Spectrophotometry, which determines the absorption or transmission of UV/VIS light by a sample. A light wavelength of 600 nm is known to be adequate for characterizing the degree of clarity of cosmetic compositions. Typically, it is best to follow the specific instructions relating the specific spectrophotometer being used. In general, the procedure for measuring percent transmittance starts by setting the spectrophotometer to the 600 nm. Then a calibration "blank" is run to calibrate the readout to 100 percent transmittance. The test sample is then placed in a cuvette designed to fit the specific spectrophotometer and the percent transmittance is measured by the spectrophotometer at 600 nm.

It is preferred if the multi-phase product has one phase that is visually clear. More preferred is a product having one visually clear phase and one opaque phase.

It is preferable if the differing phases of the composition are co-extruded.

Personal care compositions according to the invention comprise a polyacrylate-1 Crosspolymer. Poly-acrylate-1 Crosspolymer is a copolymer of one or more simple esters of acrylic or methacrylic acid, C1-4 dialkylamino C1-6 alkyl methacrylate, PEG/PPG-30/5 allyl ether, PEG 20-25 C10-30 alkyl ether methacrylate, hydroxyl C2-6 alkyl methacrylate crosslinked with ethylene glycol dimethacrylate. An especially preferred polyacrylate-1 Crosspolymer is Carbopol aqua CC (ex Lubrizol/Noveon).

Compositions of the invention are usually aqueous. Each phase comprising polyacrylate-1 crosspolymer further comprises water. The weight ratio of water to polyacrylate-1 crosspolymer is from 6:1 to 5:1.

The compositions of the invention comprise a lamellar conditioning phase. Non-limiting examples of additives that promote thickening via lamellar structuring of surfactants for use in the personal care composition include fatty amides, fatty alcohols, fatty acid or ester derivatives thereof, electrolytes, and mixtures thereof. Examples of fatty acids which my be used are C10-C22 acids such as the following: lauric acid, oleic acid, isostearic acid, linoleic acid, linolenic acid, ricinoleic acid, elaidic acid, arichidonic acid, myristoleic acid, palmitoleic acid, and the like. Ester derivatives include propylene glycol, isostearate, propylene glycol oleate, glyceryl isostearate, glyceryl oleate, polyglyoeryl diisostearate, and the like.

Other phases may include alternative thickeners. Polymeric thickeners for use in the personal care composition include Acrylates/Vinyl Isodecanoate Crosspolymer (Stabylen 30 from 3V), Acrylates/C10-30 Alkyl Acrylate Crosspolymer (Pemulen TR1 and TR2), Carbomers (Aqua SF-1), Ammonium Acryloyldimethyltaurate/VP Copolymer (Aristoflex AVC from Clariant), Ammonium Acryloyldimethyltaurate/Beheneth-25 Methacrylate Crosspolymer (Aristoflex HMB from Clariant), Acrylates/Ceteth-20 Itaconate Copolymer (Structure 3001 from National Starch), Polyacrylamide (Sepigel 305 from SEPPIC), acrylates/aminoacrylates/CD-30 alkyl PEG-20 itaconate copolymer (Structure Plus from National Starch), non-ionic thickener, (Aculyn 46 from Rohm and Haas), or mixtures thereof.

A further group of polymeric thickeners include cellulosic gel, hydroxypropyl starch phosphate (Structure XL from National Starch), polyvinyl alcohol, synthetic and natural gums and thickeners such as xanthan gum (Ketrol CG-T from CP Kelco), succinoglycan (Rheozan from Rhodia), gellum gum, pectin, alginates, starches including pregelatinized starches, modified starches, or mixtures thereof, Non-limiting examples of additives that promote thickening via lamellar structuring of surfactants for use in the personal care composition include fatty amides, fatty alcohols, fatty acid or ester derivatives thereof, electrolytes, and mixtures thereof. Examples of fatty acids which may be used are C10-C22 acids such as the following: lauric acid, oleic acid, isostearic acid, linoleic acid, linolenic acid, ricinoleic acid, elaidic acid, arichidonic acid, myristoleic acid, palmitoleic acid, and the like. Ester derivatives include propylene glycol, isostearate, propylene glycol oleate, glyceryl isostearate, glyceryl oleate, polyglyceryl diisostearate, and the like.

Non-limiting examples of organic crystalline thickeners for use in the personal care composition include ethylene glycol esters of fatty acids preferably having from about 16 to about 22 carbon atoms. Other long chain acyl derivatives include long chain esters of long chain fatty acids (e.g., stearyl stearate, cetyl palmitate, etc.); long chain esters of long chain alkanol amides (e.g., stearamide diethanolamide distearate, stearamide monoethanolamide stearate); and glyceryl esters (e. g., glyceryl distearate, trihydroxystearin, tribehenin) a commercial example of which is Thixin R available from Rheox, Inc. Other suitable thickeners are alkyl (C16 to C22) dimethyl amide oxides such as stearyl dimethyl amine oxide. Also useful herein are long chain acyl derivatives, ethylene glycol esters of long chain carboxylic acids, long chain amine oxides, and alkanol amides of long chain carboxylic acids. Inorganic water thickeners for use in the personal care composition include silicas, clays such as a synthetic silicates (Laponite XLG and Laponite XLS from Southern Clay), or mixtures thereof.

Suitable cationic conditioning agents for use in compositions of the invention, especially in the lamellar conditioning phase, are amino or quaternary ammonium hydrophilic moieties which are positively charged when dissolved in the composition.

Preferred cationic conditioning agents are quaternary ammonium cationic surfactants corresponding to the following general formula:

[N(R¹)(R²)(R³)(R⁴)]⁺ (X)⁻

in which R¹, R², R³, and R⁴ are each independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

In a suitable class of cationic surfactant of general formula (I), R¹ and R² are each independently selected from C₁₆ to C₂₂ hydrocarbyl chains comprising at least one ester linkage in both R¹ and R², and R³ and R⁴ are each independently selected from CH₃ and CH₂CH₂OH.

In another suitable class of cationic surfactant of general formula (I), R¹ and R² are each independently selected from C₁₆ to C₂₂ saturated or unsaturated, preferably saturated, chains, and R³ and R⁴ are each independently selected from CH₃ and CH₂CH₂OH, preferably CH₃.

In a preferred class of cationic surfactant of general formula (I), R¹ is a C₁₆ to C₂₂ alkyl chain and R², R³ and R⁴ are each independently selected from CH₃ and CH₂CH₂OH, preferably CH₃.

Specific examples of suitable quaternary ammonium cationic surfactants of general formula (I) are cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, dipalmitoylethyldimethylammonium chloride, PEG-2 oleylammonium chloride and salts of these, where the chloride is replaced by halogen, (e.g., bromide), acetate, citrate, lactate, glycolate, phosphate nitrate, sulphate, or alkylsulphate.

Particularly preferred quaternary ammonium cationic surfactants for use in the invention are cetyltrimethylammonium chloride, available commercially, for example as GENAMIN CTAC, ex Hoechst Celanese and Arquad 16/29 supplied by Akzo Nobel, and behenyltrimethylammonium chloride (BTAC) such as Genamin KDM-P supplied by Clariant.

Mixtures of any of the foregoing materials may also be suitable.

Salts of primary, secondary, and tertiary fatty amines are also suitable cationic surfactants for use in the invention. The alkyl groups of such amines preferably have from about 12 to about 22 carbon atoms, and can be substituted or unsubstituted. These amines are typically used in combination with an acid to provide the cationic species.

A preferred class of amine corresponds to the following general formula:

R¹ - C(O) - N(H) - R² - N(R³) (R⁴)

in which R¹ is a fatty acid chain containing from 12 to 22 carbon atoms, R² is an alkylene group containing from one to four carbon atoms, and R³ and R⁴ are, independently, an alkyl group having from one to four carbon atoms.

Specific examples of suitable materials of general formula (II) are stearamidopropyIdimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and diethylaminoethylstearamide.

Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

Particularly preferred is stearamidopropyldimethylamine.

Mixtures of any of the foregoing materials may also be suitable.

The acid used to provide the cationic species can be any organic acid or mineral acid of sufficient acid strength to neutralise a free amine nitrogen. Such acids include hydrochloric acid, sulphuric acid, nitric acid, phosphoric acid, lactic acid, citric acid, tartaric acid, acetic acid, gluconic acid, glycolic acid and propionic acid, or combinations thereof. In general, a sufficient amount of acid is added to neutralise the amidoamine compound and to adjust the final pH of the composition to within a range of from about 2.5 to about 6, preferably in a pH range of from about 3 to about 5. The molar ratio of protonatable amine groups to H⁺ from the acid is preferably from about 1:0.3 to 1:1.2, and more preferably from about 1:0.5 to about 1:1.1.

Mixtures of any of the above-described cationic surfactants may also be suitable.

In the composition of the invention, the level of cationic surfactant preferably ranges from 0.1 to 10%, more preferably 0.2 to 5%, most preferably 0.25 to 4% by total weight of cationic surfactant based on the total weight of the composition.

Compositions of the invention preferably contain electrolytes. The electrolytes are preferably in the phase comprising the polyacrylate-1 cross polymer. Preferred electrolytes are metal and ammononium salts of carbonate and sulphate. Particularly preferred is ammonium carbonate.

Preferably the level of electrolyte in the total composition is from 0.01 to 5 wt%, more preferably from 0.05 to 1 wt%.

Compositions of the invention comprise an organic acid, glycolic acid.

It is preferred if the weight ratio of polyacrylate crosspolymer to glycolic acid is from 4:1 to 1:1, more preferably from 3:1 to 1:1.

Compositions of the invention may comprise further conditioning agents to optimise wet and dry conditioning benefits.

Particularly preferred further conditioning agents are silicone emulsions.

Suitable silicone emulsions include those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer.

A suitable method for measuring the Sauter mean droplet diameter (D_{3,2}) is by laser light scattering using an instrument such as a Malvern Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula: 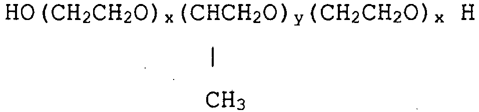 wherein the mean value of x is 4 or more and the mean value of y is 25 or more.

Another preferred form of the surface active block copolymer is according to the following formula: 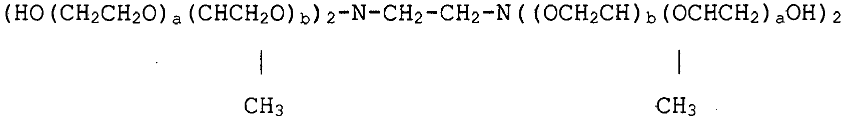 wherein the mean value of a is 2 or more and the mean value of b is 6 or more.

Mixtures of any of the above described silicone emulsions may also be used.

Silicone will generally be present in a composition of the invention at levels of from 0.05 to 10%, preferably 0.05 to 5%, more preferably from 0.5 to 2% by total weight of silicone based on the total weight of the composition.

If the product is a styling product it is preferred if a styling polymer is present.

The hair styling polymer if present is preferably present in the compositions of the invention in an amount of from 0.001% to 10% by weight, more preferably from 0.1% to 10% by weight, such as from 1% to 8% by weight.

Hair styling polymers are well known. Suitable hair styling polymers include commercially available polymers that contain moieties that render the polymers cationic, anionic, amphoteric or nonionic in nature. Suitable hair styling polymers include, for example, block and graft copolymers. The polymers may be synthetic or naturally derived.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The final product form of hair treatment compositions according to the invention may suitably be, for example, conditioners, mousses, gels, waxes or lotions. Particularly preferred product forms are conditioners (leave-in and rinse-off) and hair leave in hair treatment products.

### Examples

The invention will now be further illustrated by the following, non-limiting Examples.

A number illustrates examples of the invention; a letter illustrates Comparative Examples.

All percentages quoted are by weight based on total weight unless otherwise stated.

### Example 1

**(one opaque and one transparent phase)**

| | **Raw Material** | **Supplier** | **Activity w/w %** | **Concentration w/w %** |
|---|---|---|---|---|
| **Phase 1** | | | | |
| Aqua | Water | - | 100 | 41.705 |
| Lactic Acid | Purac HS 88 | Purac | 88 | 0.22 |
| Searamidopropyl Dimethylamine | Lexamine S13 | Inolex | 100 | 0.75 |
| Behentrimonium Chloride | Genamin BTLF | Clariant | 70 | 0.75 |
| Petrolatum | Petrolatum G 2212 | Sonneborn | 100 | 0.1 |
| Liquidum Paraffinum | White Mineral Oil | Sonneborn | 100 | 0.15 |
| Cetyl/Stearyl Alcohol | Lanette S3 | Cognis | 100 | 3 |
| PEG-180M | Polyox ™ | Amercol | 100 | 0.01 |
| | WSR 308 | | | |
| Glycerin | Glycerin USP | Fluka | 100 | 0.5 |
| Potassium Chloride | Potassium Cloride, USP/Fcc | Sigma | 99 | 0.05 |
| Disodium EDTA | Disodium EDTA | Surfachem | 100 | 0.05 |
| Dimethyl Methyl (aminoethylaminoisobu tyl) Siloxane | DC 1785 | Dow Corning | 60 | 2.145 |
| Parfum | Hyperion 85J | Givaudan | 100 | 0.5 |
| Methylchloroisothiazo linone and Methylisothiazolinone | Kathon CG | Rohm and Haas | 1.5 | 0.02 |
| DMDM Hydantoin | Glydant | Lonza | 55 | 0.05 |

| **Phase 2** | | | | |
|---|---|---|---|---|
| Aqua | Water | | 100 | 39.28 |
| Polyacrylate-1 Crosspolymer | Carbopol Aqua CC | Lubrizol | 20 | 7.5 |
| Glycolic Acid | Glycolic Acid | Sigma | 20 | 2.25 |
| Trehalose | Trehalose | Sigma | 100 | 0.6 |
| Ammonium Carbonate | Ammonium Carbonate | Sigma | 100 | 0.15 |
| Adipic Acid | Adipic Acid | Sigma | 100 | 0.15 |
| DMDM Hydantoin | Glydant | Lonza | 55 | 0.05 |
| Methylchloroisothiazo linone and Methylisothiazolinone | Kathon CG | Rohm and Haas | 1.5 | 0.02 |

### Example 2

**(one opaque and one shimmering phase)**

| | **Raw Material** | **Supplier** | **Activity w/w %** | **Concentration w/w %** |
|---|---|---|---|---|
| **Phase 1** | | | | |
| Aqua | Water | - | 100 | 41.705 |
| Lactic Acid | Purac HS 88 | Purac | 88 | 0.22 |
| Searamidopropyl Dimethylamine | Lexamine S13 | Inolex | 100 | 0.75 |
| Behentrimonium Chloride | Genamin BTLF | Clariant | 70 | 0.75 |
| Petrolatum | Petrolatum G 2212 | Sonneborn | 100 | 0.1 |
| Liquidum Paraffinum | White Mineral Oil | Sonneborn | 100 | 0.15 |
| Cetyl/Stearyl Alcohol | Lanette S3 | Cognis | 100 | 3 |
| PEG-180M | Polyox ™ WSR 308 | Amercol | 100 | 0.01 |
| Glycerin | Glycerin USP | Fluka | 100 | 0.5 |
| Potassium Chloride | Potassium Cloride, USP/Fcc | Sigma | 99 | 0.05 |
| Disodium EDTA | Disodium EDTA | Surfachem | 100 | 0.05 |
| Dimethyl Methyl (aminoethylaminoisobu tyl) Siloxane | DC 1785 | Dow Corning | 60 | 2.145 |
| Parfum | Hyperion 85J | Givaudan | 100 | 0.5 |
| Methylchloroisothiazo linone and Methylisothiazolinone | Kathon CG | Rohm and Haas | 1.5 | 0.02 |
| DMDM Hydantoin | Glydant | Lonza | 55 | 0.05 |

| **Phase 2** | | | | |
|---|---|---|---|---|
| Aqua | Water | - | 100 | 38.98 |
| Polyacrylate-1 Crosspolymer | Carbopol Aqua CC | Lubrizol | 20 | 7.5 |
| Glycolic Acid | Glycolic Acid | Sigma | 20 | 2.25 |
| Trehalose | Trehalose | Sigma | 100 | 0.6 |
| Ammonium Carbonate | Ammonium Carbonate | Sigma | 100 | 0.15 |
| Adipic Acid | Adipic Acid | Sigma | 100 | 0.15 |
| DMDM Hydantoin | Glydant | Lonza | 55 | 0.05 |
| Methylchloroisothiazo linone and Methylisothiazolinone | Kathon CG | Rohm and Haas | 1.5 | 0.02 |
| | Ronstar Golden Sparks | Merck | 100 | 0.3 |

### Example A

| | **Raw Material** | **Supplier** | **Activity w/w %** | **Concentration w/w %** |
|---|---|---|---|---|
| **Phase 1** | | | | |
| Aqua | Water | - | 100 | 41.705 |
| Lactic Acid | Purac HS 88 | Purac | 88 | 0.22 |
| Searamidopropyl Dimethylamine | Lexamine S13 | Inolex | 100 | 0.75 |
| Behentrimonium Chloride | Genamin BTLF | Clariant | 70 | 0.75 |
| Petrolatum | Petrolatum G 2212 | Sonneborn | 100 | 0.1 |
| Liquidum Paraffinum | White | Sonneborn | 100 | 0.15 |
| Cetyl/Stearyl Alcohol | Mineral Oil Lanette S3 | Cognis | 100 | 3 |
| PEG-180M | Polyox ™ WSR 308 | Amercol | 100 | 0.01 |
| Glycerin | Glycerin USP | Fluka | 100 | 0.5 |
| Potassium Chloride | Potassium Cloride, USP/Fcc | Sigma | 99 | 0.05 |
| Disodium EDTA | Disodium EDTA | Surfachem | 100 | 0.05 |
| Dimethyl Methyl (aminoethylaminoisobu tyl) Siloxane | DC 1785 | Dow Corning | 60 | 2.145 |
| Parfum | Hyperion 85J | Givaudan | 100 | 0.5 |
| Methylchloroisothiazo linone and Methylisothiazolinone | Kathon CG | Rohm and Haas | 1.5 | 0.02 |
| DMDM Hydantoin | Glydant | Lonza | 55 | 0.05 |

| **Phase 2** | | | | |
|---|---|---|---|---|
| Aqua | Water | - | 100 | 48.53 |
| Polyquaternium 37 | Ultragel 300 | Cognis | 100 | 0.5 |
| Trehalose | Trehalose | Sigma | 100 | 0.6 |
| Ammonium Carbonate | Ammonium Carbonate | Sigma | 100 | 0.15 |
| Adipic Acid | Adipic Acid | Sigma | 100 | 0.15 |
| DMDM Hydantoin | Glydant | Lonza | 55 | 0.05 |
| Methylchloroisothiazo linone and Methylisothiazolinone | Kathon CG | Rohm and Haas | 1.5 | 0.02 |

### Example B

| **Phase 1** | | | | |
|---|---|---|---|---|
| Aqua | Water | - | 100 | 41.705 |
| Lactic Acid | Purac HS 88 | Purac | 88 | 0.22 |
| Searamidopropyl Dimethylamine | Lexamine S13 | Inolex | 100 | 0.75 |
| Behentrimonium Chloride | Genamin BTLF | Clariant | 70 | 0.75 |
| Petrolatum | Petrolatum G 2212 | Sonneborn | 100 | 0.1 |
| Liquidum Paraffinum | White Mineral Oil | Sonneborn | 100 | 0.15 |
| Cetyl/Stearyl Alcohol | Lanette S3 | Cognis | 100 | 3 |
| PEG-180M | Polyox ™ WSR 308 | Amercol | 100 | 0.01 |
| Glycerin | Glycerin USP | Fluka | 100 | 0.5 |
| Potassium Chloride | Potassium Cloride, USP/Fcc | Sigma | 99 | 0.05 |
| Disodium EDTA | Disodium EDTA | Surfachem | 100 | 0.05 |
| Dimethyl Methyl (aminoethylaminoisobu tyl) Siloxane | DC 1785 | Dow Corning | 60 | 2.145 |
| Parfum | Hyperion 85J | Givaudan | 100 | 0.5 |
| Methylchloroisothiazo linone and Methylisothiazolinone | Kathon CG | Rohm and Haas | 1.5 | 0.02 |
| DMDM Hydantoin | Glydant | Lonza | 55 | 0.05 |

| **Phase 2** | | | | |
|---|---|---|---|---|
| Aqua | Water | - | 100 | 48.43 |
| Cellulose, 2-hydroxyethyl ether | Natrosol 250 HHR | Hercules | 100 | 1.5 |
| DMDM Hydantoin | Glydant | Lonza | 55 | 0.05 |
| Methylchloroisothiazo linone and Methylisothiazolinone | Kathon CG | Rohm and Haas | 1.5 | 0.02 |

### Example 3 (comparative example)

| **Phase 1** | | | | |
|---|---|---|---|---|
| Aqua | Water | - | 100 | 41.705 |
| Lactic Acid | Purac HS 88 | Purac | 88 | 0.22 |
| Searamidopropyl Dimethylamine | Lexamine S13 | Inolex | 100 | 0.75 |
| Behentrimonium Chloride | Genamin BTLF | Clariant | 70 | 0.75 |
| Petrolatum | Petrolatum G 2212 | Sonneborn | 100 | 0.1 |
| Liquidum Paraffinum | White Mineral Oil | Sonneborn | 100 | 0.15 |
| Cetyl/Stearyl Alcohol | Lanette S3 | Cognis | 100 | 3 |
| PEG-180M | Polyox ™ WSR 308 | Amercol | 100 | 0.01 |
| Glycerin | Glycerin USP | Fluka | 100 | 0.5 |
| Potassium Chloride | Potassium Cloride, USP/Fcc | Sigma | 99 | 0.05 |
| Disodium EDTA | Disodium EDTA | Surfachem | 100 | 0.05 |
| Dimethyl Methyl (aminoethylaminoisobu tyl) Siloxane | DC 1785 | Dow Corning | 60 | 2.145 |
| Parfum | Hyperion 85J | Givaudan | 100 | 0.5 |
| Methylchloroisothiazo linone and Methylisothiazolinone | Kathon CG | Rohm and Haas | 1.5 | 0.02 |
| DMDM Hydantoin | Glydant | Lonza | 55 | 0.05 |

| **Phase 2** | | | | |
|---|---|---|---|---|
| Aqua | Water | - | 100 | 40.53 |
| Polyacrylate-1 Crosspolymer | Carbopol Aqua CC | Lubrizol | 20 | 6.25 |
| Glycolic Acid | Glycolic Acid | Sigma | 20 | 2.25 |
| Trehalose | Trehalose | Sigma | 100 | 0.6 |
| Ammonium Carbonate | Ammonium Carbonate | Sigma | 100 | 0.15 |
| Adipic Acid | Adipic Acid | Sigma | 100 | 0.15 |
| DMDM Hydantoin | Glydant | Lonza | 55 | 0.05 |
| Methylchloroisothiazo linone and Methylisothiazolinone | Kathon CG | Rohm and Haas | 1.5 | 0.02 |

### Example 4 (comparative example)

| | **Raw Material** | **Supplier** | **Activity w/w %** | **Concentration w/w %** |
|---|---|---|---|---|
| **Phase 1** | | | | |
| Aqua | Water | - | 100 | 41.705 |
| Lactic Acid | Purac HS 88 | Purac | 88 | 0.22 |
| Searamidopropyl Dimethylamine | Lexamine S13 | Inolex | 100 | 0.75 |
| Behentrimonium Chloride | Genamin BTLF | Clariant | 70 | 0.75 |
| Petrolatum | Petrolatum G 2212 | Sonneborn | 100 | 0.1 |
| Liquidum Paraffinum | White Mineral Oil | Sonneborn | 100 | 0.15 |
| Cetyl/Stearyl Alcohol | Lanette S3 | Cognis | 100 | 3 |
| PEG-180M | Polyox ™ WSR 308 | Amercol | 100 | 0.01 |
| Glycerin | Glycerin USP | Fluka | 100 | 0.5 |
| Potassium Chloride | Potassium Cloride, USP/Fcc | Sigma | 99 | 0.05 |
| Disodium EDTA | Disodium EDTA | Surfachem | 100 | 0.05 |
| Dimethyl Methyl (aminoethylaminoisobu tyl) Siloxane | DC 1785 | Dow Corning | 60 | 2.145 |
| Parfum | Hyperion 85J | Givaudan | 100 | 0.5 |
| Methylchloroisothiazo linone and Methylisothiazolinone | Kathon CG | Rohm and Haas | 1.5 | 0.02 |
| DMDM Hydantoin | Glydant | Lonza | 55 | 0.05 |

| **Phase 2** | | | | |
|---|---|---|---|---|
| Aqua | Water | - | 100 | 39.53 |
| Polyacrylate-1 Crosspolymer | Carbopol Aqua CC | Lubrizol | 20 | 6.25 |
| Lactic Acid | Purac HS 88 | Purac | 88 | 3.25 |
| Trehalose | Trehalose | Sigma | 100 | 0.6 |
| Ammonium Carbonate | Ammonium Carbonate | Sigma | 100 | 0.15 |
| Adipic Acid | Adipic Acid | Sigma | 100 | 0.15 |
| DMDM Hydantoin | Glydant | Lonza | 55 | 0.05 |
| Methylchloroisothiazo linone and Methylisothiazolinone | Kathon CG | Rohm and Haas | 1.5 | 0.02 |

In all examples Phase 1 is a cationic conditioning formula (a conditioning gel phase). In all examples the rheology modifiers used in Phase 1 are either cationic or non-ionic.

No comparative examples using anionic rheology modifiers are due to incompatibility of both phases.

These examples demonstrate the advantage of Carbopol Aqua CC over other cationic rheology modifiers.

Examples 1 and 2 illustrate two phase formulations where one of the phases is white and opaque while the other phase is yellow and transparent (Example 1) or golden shimmering (Example 2). When co-packed in a single airless pump (the two phases are in prolonged contact) the product remains stable. On discharge from the pack the two phases mix well together giving a smooth texture.

In Example A attempt was made to use Ultragel 300 to formulate a transparent phase compatible with Phase 1, however the result was a non-transparent, low viscosity mixture. The viscosity of the mixture was too low to allow suspension of any design made from Phase 1.

In Example B attempt was made to use Natrosol 250 HHR to formulate a transparent phase compatible with Phase 1. The Natrosol 250 HHR gave transparent gel which when co-extruded in a single pack with Phase 1 showed no instability at the interfaces, the transparent gel was able to suspend a design made by Phase 1 and on discharge from the pack the two phases mix well together giving a smooth texture. However when stored at 45°C the viscosity of Phase 2 decreased to an extent that lead to loss of suspension properties under those conditions. It is not unlikely during transportation or storage the product to be exposed to so high temperatures which would have lead to loss of the aesthetic properties of the formulation.

Example 3 uses the same ingredients as Example 1 but the level of Carbopol Aqua CC is lower. Example 3 did not have such good long term storage stability at high temperature as Examples 1 and 2.

Example 4 is similar to Example 1 but uses lactic acid instead of glycolic acid to neutralise the Carbopol Aqua CC. The compositions have comparable pH and similar zero shear rate viscosity to allow suspension of another phase under relatively high temperature conditions, i.e. 45°C. However Example 4 did not have such good long term storage stability at high temperature as Examples 1 and 2.

## Claims

1. A multi-phase personal care composition comprising at least two visually distinct phases in physical contact with one another **characterised in that** a) at least one phase comprises i) water and polyacrylate-1 crosspolymer at a ratio of 6:1 to 5:1 and ii) glycolic acid; and b) one phase is a lamellar conditioning phase, comprising a cationic conditioning ingredient.

2. A multi-phase personal care composition according to claim 1 or claim 2 in which at least one phase is visually clear.

3. A multi-phase personal care composition according to any preceding claim in which at least one phase is opaque.

4. A multi-phase care personal care composition according to any preceding claim in which at least one phase comprises electrolyte.

5. A multi-phase composition according to claim 5 in which the electrolyte and polyacrylate-1 crosspolymer are in the same phase.

6. A multi-phase personal care composition according to any preceding claim in which the ratio of polyacrylate crosspolymer to glycolic acid is from 3:1 to 1:1.

7. A multi-phase personal care composition according to any preceding claim for use on hair.

8. A personal care composition according to any preceding claim in which the differing phases of the composition are co-extruded.

9. A pack comprising a multi-phase composition as claimed in any one of claims 1 to 9.

## Patentansprüche

1. Körperpflegezusammensetzung mit mehreren Phasen, die mindestens zwei visuell unterschiedliche Phasen in körperlichem Kontakt miteinander aufweist,
**dadurch gekennzeichnet, dass** a) mindestens eine Phase i) Wasser und ein Polyacrylat-1-Crosspolymer in einem Verhältnis von 6:1 bis 5:1 und ii) Glycolsäure aufweist; und b) eine Phase eine lamellare konditionierende Phase ist, die einen kationischen konditionieren Bestandteil aufweist.

2. Körperpflegezusammensetzung mit mehreren Phasen nach Anspruch 1 oder 2,
wobei zumindest eine Phase durchsichtig ist.

3. Körperpflegezusammensetzung mit mehreren Phasen nach einem der vorstehenden Ansprüche,
wobei zumindest eine Phase undurchsichtig ist.

4. Körperpflegezusammensetzung mit mehreren Phasen nach einem der vorstehenden Ansprüche,
wobei zumindest eine Phase einen Elektrolyt aufweist.

5. Körperpflegezusammensetzung mit mehreren Phasen nach Anspruch 5,
wobei der Elektrolyt und das Polyacrylat-1-Crosspolymer in der gleichen Phase vorliegen.

6. Körperpflegezusammensetzung mit mehreren Phasen nach einem der vorstehenden Ansprüche,
wobei das Verhältnis zwischen Polyacrylat-Crosspolymer und Glycolsäure 3:1 bis 1:1 beträgt.

7. Körperpflegezusammensetzung mit mehreren Phasen nach einem der vorstehenden Ansprüche für die Verwendung bei Haaren.

8. Körperpflegezusammensetzung nach einem der vorstehenden Ansprüche,
wobei die verschiedenen Phasen der Zusammensetzung coextrudiert sind.

9. Packung,
die eine Zusammensetzung mit mehreren Phasen nach einem der Ansprüche 1 bis 9 aufweist.

## Revendications

1. Composition multiphase pour le soin de la personne comprenant au moins deux phases visuellement distinctes en contact physique l'une avec l'autre **caractérisée en ce que** a) au moins une phase comprend i) de l'eau et un polymère réticulé de polyacrylate-1 à un rapport de 6:1 à 5:1 et ii) de l'acide glycolique ; et b) une phase est une phase de conditionnement lamellaire, comprenant un ingrédient de conditionnement cationique.

2. Composition multiphase pour le soin de la personne selon la revendication 1 ou la revendication 2, dans laquelle au moins une phase est visuellement transparente.

3. Composition multiphase pour le soin de la personne selon l'une quelconque des revendications précédentes, dans laquelle au moins une phase est opaque.

4. Composition multiphase pour le soin de la personne selon l'une quelconque des revendications précédentes, dans laquelle au moins une phase comprend un électrolyte.

5. Composition multiphase selon la revendication 5 dans laquelle l'électrolyte et le polymère réticulé de polyacrylate-1 sont dans la même phase.

6. Composition multiphase pour le soin de la personne selon l'une quelconque des revendications précédentes dans laquelle le rapport de polymère réticulé de polyacrylate à l'acide glycolique est de 3:1 à 1:1.

7. Composition multiphase pour le soin de la personne selon l'une quelconque des revendications précédentes pour une utilisation sur des cheveux.

8. Composition pour le soin de la personne selon l'une quelconque des revendications précédentes, dans laquelle les phases différentes de la composition sont co-extrudées.

9. Pack comprenant une composition multiphase selon l'une quelconque des revendications 1 à 9.
